# EUROPÄISCHE PATENTSCHRIFT

(11) **EP 1 882 456 B1**
(45) Veröffentlichungstag und Bekanntmachung des Hinweises auf die Patenterteilung: **29.06.2016**
(21) Anmeldenummer: 07014257.5
(22) Anmeldetag: 20.07.2007
(51) Int. Cl.: A61B 17/34, A61B 17/17

(54) **Teilzielvorrichtung zum Anzielen einer arthroskopischen Operationsstelle für einen medizinischen Eingriff**
Partial target device for targeting an arthroscopic operation point for surgery
Dispositif à objectif partiel destiné à viser une position d'opération arthroscopique pour une opération médicale

(30) Priorität: 27.07.2006 DE 102006035579
(43) Veröffentlichungstag der Anmeldung: 30.01.2008
(73) Patentinhaber: Karl Storz GmbH & Co. KG, 78532 Tuttlingen (DE)
(72) Erfinder: Dienst, Michael, 66424 Homburg (DE); Berberich, Sascha, 78532 Tuttlingen (DE)
(74) Vertreter: Witte, Weller & Partner Patentanwälte mbB

(56) Entgegenhaltungen:
- DE-A1- 3 436 253
- GB-A- 2 230 453
- US-A- 5 688 284
- US-A1- 2004 193 172

## Beschreibung

Die Erfindung betrifft eine Teilzielvorrichtung zum Anzielen einer arthroskopischen Operationsstelle für einen medizinischen Eingriff, mit einem kreisabschnittförmigen Bogen, mit einem entlang des kreisabschnittförmigen Bogens bewegbaren, sich radial erstreckenden ersten Zielstab, wobei an dem kreisabschnittförmigen Bogen eine Verbindungsvorrichtung angeordnet ist, mittels der ein einen Schaft aufweisendes medizinisches Instrument an die Teilzielvorrichtung anbringbar ist, und zwar derart, dass der Schaft als zweiter, radialer sich zum ersten Zielstab erstreckender Zielstab ausgerichtet ist, wobei die Verbindungsvorrichtung derart ausgebildet ist, dass diese an eine bereits vorhandene Verbindungsstelle des medizinischen Instrumentes anbringbar ist.

Aus der GB-A-2 230 453 ist eine Zielvorrichtung zum Anzielen einer arthroskopischen Operationsstelle für einen medizinischen Eingriff bekannt. Diese Zielvorrichtung weist einen kreisabschnittförmigen Bogen auf, entlang dem ein sich radial erstreckender erster Zielstab bewegbar ist. An dem kreisabschnittförmigen Bogen ist ferner eine Verbindungsvorrichtung angeordnet, die einen vorspringenden Zahn aufweist, mittels dem ein an seiner Außenseite mit einer entsprechenden Verzahnung versehenes medizinisches Instrument an den Bogen anbringbar ist. Das so angebrachte medizinische Instrument erstreckt sich ebenfalls radial und ist als zweiter Zielstab ausgebildet. Ist das Instrument an dem Bogen montiert, ist die Vorrichtung als komplette Zielvorrichtung einsetzbar.

Aus der DE-A-3436253 ist eine Zielvorrichtung offenbart, die zum Führen eines Knochenbohrers geeignet ist. Hierzu besteht diese Zielvorrichtung aus einem kreisabschnittförmigen Bogen, an dem ein beweglich angeordneter Zielstab sowie eine entsprechend beweglich angeordnete Führungshülse befestigt ist. Durch diese Führungshülse ist es möglich, einen Knochenbohrer entlang einer geraden auf einen durch den Zielstab anfixierten Punkt zu führen.

Solche Zielvorrichtungen werden im medizinischen Bereich zum Anzielen einer nicht sichtbaren Operationsstelle bei einer arthroskopischen Untersuchung oder einem chirurgischen Eingriff eingesetzt. Die Arthroskopie bzw. Gelenkspiegelung ist ein minimalinvasiver chirurgischer Eingriff, der zur Diagnose, z.B. zur Untersuchung des Zustandes von Knorpeln und Bändern, und/oder zur Therapie, z.B. der Entfernung des Meniskus, der Knorpelglättung usw., dienen kann.

Während einer arthroskopischen Untersuchung bzw. eines arthroskopischen Eingriffs wird das Gelenk von innen untersucht. Es wird durch einen kleinen Hautschnitt ein Arthroskop in den Gelenkraum eingeführt. Durch das Arthroskop, das eine Optik enthält, lassen sich Gelenkstrukturen betrachten. An das Arthroskop kann zusätzlich eine Kamera angeschlossen werden, die Bilder auf einen Motor übermittelt.

Um das Arthroskop an die gewünschte Stelle in dem Gelenk zu bringen, die für den Operateur nicht ersichtlich ist, werden derartige Zielvorrichtungen verwendet.

Die Längsachsen vom ersten und zweiten Zielstab schneiden sich in etwa im Bereich der Operationsstelle.

Bei einer Kniespiegelung wird die Spitze des ersten Zielstabes von außen an eine Seite des Knies so angelegt, dass diese in etwa an der gedachten Austrittsstelle der Verlängerung der Hülse des zweiten Zielstabes liegt.

Der ebenfalls mit dem Bogen verbundene zweite Zielstab wird dann an der gegenüberliegenden Seite angelegt. Dem Arzt wird dadurch von außen die Richtung ersichtlich, längs derer das arthroskopische Instrument durch den zweiten Zielstab in das Kniegelenk eingeführt wird. Nach gewünschter Ausrichtung werden erster und zweiter Zielstab fest an den gegenüberliegenden Seiten angelegt und über den Bogen so gehalten.

Das Instrument kann jetzt zielgerecht durch den zweiten Zielstab in das Gelenk eingeführt und der Eingriff bzw. die Untersuchung kann durchgeführt werden.

Die Zielvorrichtung bleibt während des gesamten Eingriffs am Körper. Die Zielvorrichtung ist ein sperriges Gerät, das nur zum Anzielen, aber nicht für den eigentlichen Eingriff bei der Untersuchung notwendig ist. Die sperrige Vorrichtung stört den Raum um das Gelenk. Es ist daher Aufgabe der Erfindung, hier Abhilfe zu schaffen und eine Vorrichtung zu schaffen, mittels der der Operationsort genau angezielt werden kann, die aber bei dem eigentlichen Eingriff bzw. der Untersuchung nicht stört.

Diese Aufgabe wird durch eine Teilzielvorrichtung gemäss Anspruch 1 gelöst.

Diese Maßnahme hat den Vorteil, dass die meisten medizinischen Instrumente, mit denen solche Untersuchungen oder Eingriffe durchgeführt werden, einen LUER-Anschluss, also einen Anschluss in Form eines einfachen Gewindes, der sich mit einer halben Drehung schließt und öffnet, aufweisen. Ein LUER-lock-System, das international als Standardanschluss erkannt ist, ermöglicht eine Kompatibilität der Teilzielvorrichtung mit Instrumenten verschiedener Hersteller. Diese Maßnahme hat auch den Vorteil, dass über den LUER-Anschluss der Bogen samt dem ersten Zielstab an dasjenige medizinische Instrument angebracht werden kann, mit dem die eigentliche arthroskopische Untersuchung bzw. der Eingriff durchgeführt wird.

Dadurch ist es möglich, die Zielvorrichtung als Teilzielvorrichtung auszubilden, die aus dem Bogen, dem ersten Zielstab und der Verbindungsvorrichtung zur Verbindung an einen LUER-Anschluss besteht.

Dieser Zusammenbau bildet dann eine komplette Zielvorrichtung, die sich aus dem Bogen und den beiden Zielstäben zusammensetzt. Die nunmehr komplettierte Zielvorrichtung kann, wie eingangs beschrieben, an das Gelenk angelegt werden und der zweite Zielstab, also der Schaft des medizinischen Instruments, kann zielgerecht in das Gelenk eingeführt werden. Nachdem der Schaft des medizinischen Instruments in das Gelenk eingeführt ist, kann die Zielvorrichtung wieder abgenommen werden, so dass für die Untersuchung oder den eigentlichen Eingriff nur noch das medizinische Instrument im Gelenk befindlich ist. Somit wird der Bereich um das Gelenk nicht mehr durch den sperrigen Zusammenbau durch den Bogen und den zweiten Zielstab behindert.

Es ist somit das Anzielen sehr sicher und wie gewohnt mit einer kompletten Zielvorrichtung durchzuführen. Durch die Ausbildung als Teilzielvorrichtung kann nach Anzielen der Operation- bzw. Untersuchungsstelien der Bogen und der erste Zielschaft, also die eigentlichen sperrig abstehenden störenden Bauelemente, abgenommen werden. Dadurch, dass die Verbindungsvorrichtung derart ausgebildet ist, dass sie an einen LUER-Anschluss angebracht werden kann, ist eine Verbindung mit einer üblicherweise an Instrumenten vorhandenen Verbindungsstelle einfach möglich.

Somit kann die Teilzielvorrichtung mit schon vorhandenen Instrumenten, die einen LUER-Anschluss aufweisen, angewendet werden. Somit wird vermieden, dass die medizinischen Instrumente an die Teilzielvorrichtung angepasst werden, sondern dass dieses Instrument über seinen LUER-Anschluss an das Instrument angepasst ist.

In einer weiteren Ausgestaltung der Erfindung weist die Verbindungsvorrichtung einen Stützbügel zum Abstützen am medizinischen Instrument auf.

Diese Maßnahme hat den Vorteil, dass durch eine derartige Ausgestaltung der Verbindungsvorrichtung für einen stabilen Sitz des medizinischen Instruments an der erfindungsgemäßen Teilzielvorrichtung gesorgt wird.

Das angebrachte medizinische Instrument sitzt an der erfindungsgemäßen Teilzielvorrichtung durch die zusätzliche Abstützstelle während des Anzielens einer arthroskopischen Operationsstelle kippsicher.

In einer weiteren Ausgestaltung der Erfindung ist ein erstes Ende des Stützbügels mit der Verbindungsvorrichtung fest verbunden.

Diese Maßnahme hat den Vorteil, dass der stabile Sitz des medizinischen Instruments an der erfindungsgemäßen Teilzielvorrichtung verstärkt wird.

In einer weiteren Ausgestaltung der Erfindung ist ein zweites Ende des Stützbügels am medizinischen Instrument anlegbar.

Diese Maßnahme hat den Vorteil, dass die zweite Stützstelle einfach zu bewerkstelligen ist, so dass ein einfaches Lösen der Teilzielvorrichtung von dem medizinischen Instrument nach wie vor ermöglicht wird.

In einer weiteren Ausgestaltung der Erfindung ist ein zweites Ende des Stützbügels am medizinischen Instrument formschlüssig anlegbar.

Diese Maßnahme hat den Vorteil, dass durch ein formschlüssiges Anlegen des zweiten Endes des Stützbügels am medizinischen Instrument gleichzeitig ein besonders stabiles Anbringen und ein einfaches Lösen des medizinischen Instruments von der Teilzielvorrichtung erreicht wird.

In einer weiteren Ausgestaltung der Erfindung weist die Verbindungsvorrichtung zusätzlich eine Verstellvorrichtung auf, durch die die Verbindungsvorrichtung in Richtung des Schaftes des medizinischen Instruments axial bewegbar ist.

Diese Maßnahme hat den Vorteil, dass der Schaft des an der Verbindungsvorrichtung angebrachten medizinischen Instruments entlang seiner Längsachse verschiebbar ist. Somit kann die Platzierung des Schaftes des medizinischen Instruments an dem Gelenk optimiert werden.

In einer weiteren Ausgestaltung der Erfindung beträgt ein Winkel zwischen einer Längsachse des ersten Zielstabs und einer Längsachse des Schaftes des medizinischen Instruments etwa 30° bis 80°.

Diese Maßnahme hat den Vorteil, dass in diesem Winkelbreich die Zielstäbe zum Anzielen positionierbar sind.

In einer weiteren Ausgestaltung der Erfindung ist der erste Zielstab als Hülse ausgebildet.

Diese Maßnahme hat den Vorteil, dass durch den als Hülse ausgebildeten ersten Zielstab, ein anderes medizinisches Instrument, bspw. eine Punktionsnadel, hindurchgeführt werden kann. Dies kann als zusätzliche Zielhilfe für das eigentliche medizinische Instrument dienen.

In einer weiteren Ausgestaltung der Erfindung weist der Bogen eine Winkelskala auf.

Dadurch ist es möglich, die exakte kreisabschnittförmige Winkelstellung des Schaftes des medizinischen Instruments zu der Längsachse des Zielstabs festzustellen oder zu dokumentieren.

In einer weiteren Ausgestaltung der Erfindung ist das medizinische Instrument als ein Arthroskop ausgebildet.

Diese Maßnahme hat den Vorteil, dass durch Verwenden eines Arthroskops, das eine in dem Schaft angeordnete Optik aufweist, das gesamte Gelenk von innen inspiziert und ggf. ein Eingriff durchgeführt werden kann.

An das Arthroskop können nach Abnehmen der Teilzielvorrichtung an deren LUER-Anschluss Schläuche angeschlossen werden, durch die eine sterile Flüssigkeit in das Gelenk eingebracht oder abgesaugt wird.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels im Zusammenhang mit den Zeichnungen näher beschrieben und erläutert. Es zeigen:
- Fig. 1: eine Seitenansicht einer erfindungsgemäßen Teilzielvorrichtung,
- Fig. 2: eine andere Seitenansicht der erfindungsgemäßen Vorrichtung von Fig. 1,
- Fig. 3: einen Schnitt entlang einer Längsachse einer Verstellvorrichtung,
- Fig. 4: eine der Fig. 1 vergleichbare Seitenansicht der erfindungsgemäßen Teilzielvorrichtung, wobei ein noch nicht an die erfindungsgemäße Teilzielvorrichtung angebrachtes medizinisches Instrument dargestellt ist,
- Fig. 5: eine der Fig. 4 vergleichbare Darstellung, wobei das medizinische Instrument an die Teilzielvorrichtung angebracht ist, und
- Fig. 6: eine der Fig. 5 vergleichbare Darstellung, wobei die Verstellvorrichtung in einem vorgeschobenen Zustand dargestellt ist.

Eine in den Figuren dargestellte Teilzielvorrichtung zum Anzielen einer arthroskopischen Operationsstelle für einen medizinischen Eingriff oder eine Untersuchung ist in ihrer Gesamtheit mit der Bezugsziffer 10 bezeichnet.

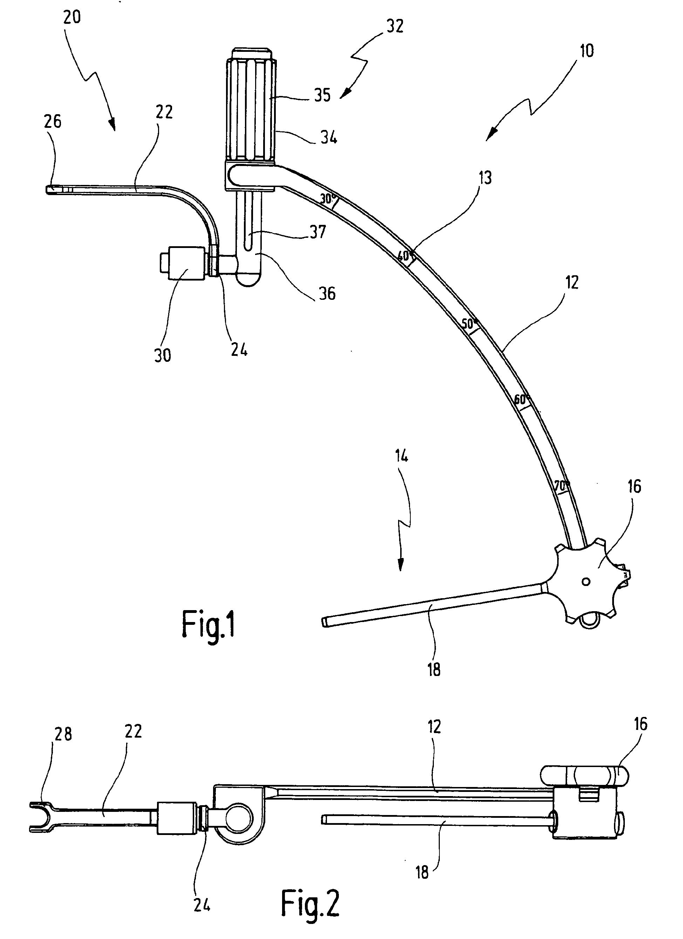

Die in Fig. 1 dargestellte Teilzielvorrichtung 10 weist einen kreisabschnittförmigen Bogen 12 auf. Der Bogen 12 weist eine Winkelskala 13 auf.

An dem kreisabschnittförmigen Bogen 12 ist ein erster, sich radial erstreckender Zielstab 14 mit einem Aufsatz 16 angeordnet.

Der erste Zielstab 14 kann mittels des Aufsatzes 16 längs des kreisabschnittförmigen Bogens 12 hin- und herverschoben werden und erstreckt sich in allen Schiebepositionen radial zum Kreisbogen.

Der Aufsatz 16 weist eine hier nicht dargestellte Vorrichtung auf, die dazu dient, diesen Verschiebevorgang zu ermöglichen und den Zielstab 14 in einer bestimmten Position an dem kreisabschnittförmigen Bogen 12 zu fixieren.

In diesem in Fig. 1 dargestellten Ausführungsbeispiel ist der erste Zielstab 14 als eine Hülse 18 abgebildet, die eine hier nicht dargestellte durch diese hindurchgehende Bohrung aufweist. Durch die Bohrung der Hülse 18 kann bspw. eine Punktionsnadel durchgeführt werden, um in das Gelenk eingestochen zu werden.

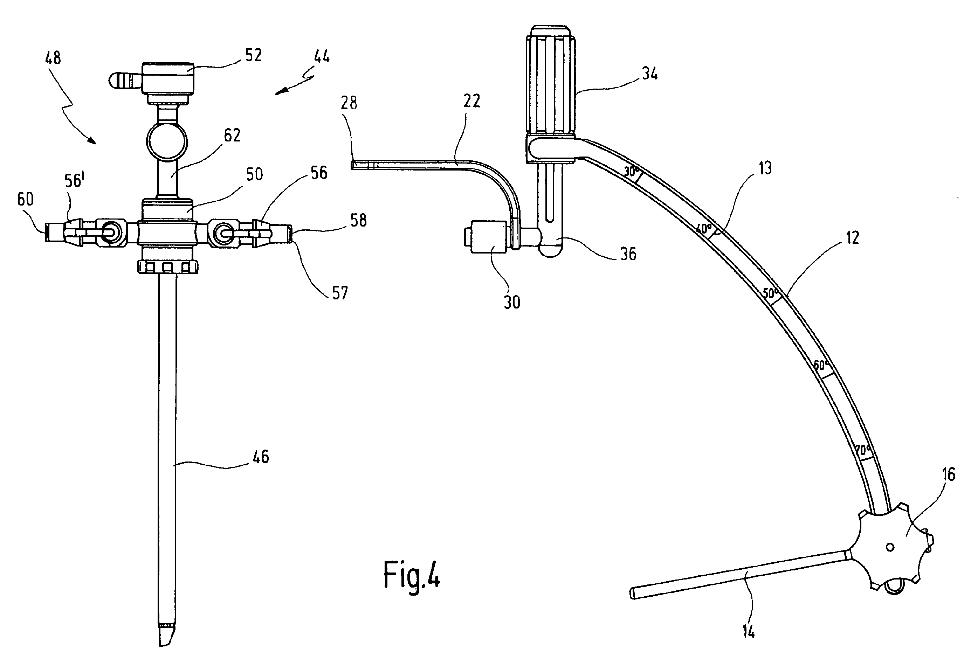

An dem kreisabschnittförmigen Bogen 12 ist eine Verbindungsvorrichtung 20 angeordnet, die dazu dient, die erfindungsgemäße Teilzielvorrichtung 10 mit einem medizinischen Instrument 42, das in Figuren 4 bis 6 dargestellt ist, zu verbinden.

Die Verbindungsvorrichtung 20 weist einen Stützbügel 22 zum Abstützen an dem medizinischen Instrument 42 auf. Ein erstes Ende 24 des Stützbügels 22 ist mit der Verbindungsvorrichtung 20 fest verbunden.

Ein zweites Ende 26 des Stützbügels 22, das zum formschlüssigen Anlegen an dem medizinischen Instrument 42 dient, ist als eine Gabel 28 ausgebildet, wie es insbesondere aus der Darstellung von Fig. 2 ersichtlich ist.

Die Verbindungsvorrichtung 20 weist einen Anschluss 30 auf, der derart ausgebildet ist, dass er an eine bereits vorhandene Verbindungsstelle des medizinischen Instruments 42 anbringbar ist. Die Ausgestaltung der Verbindungsstelle des medizinischen Instruments 42 wird in Zusammenhang mit den Figuren 4 bis 6 näher erläutert.

Die Verbindungsvorrichtung 20 weist zusätzlich eine Verstellvorrichtung 32 auf, durch die die Verbindungsvorrichtung 20 radial in der Ebene des Bogens 12 bewegbar ist.

Die Verstellvorrichtung 32, die an einem Ende des kreisabschnittförmigen Bogens 12 angeordnet ist, weist einen stabförmigen drehbaren Griff 34 auf.

In der Außenseite des Griffs 34 ist ein Kerbmuster eingeschnitten, so dass die Verstellvorrichtung 32 fest und sicher von einer menschlichen Hand ergriffen werden kann.

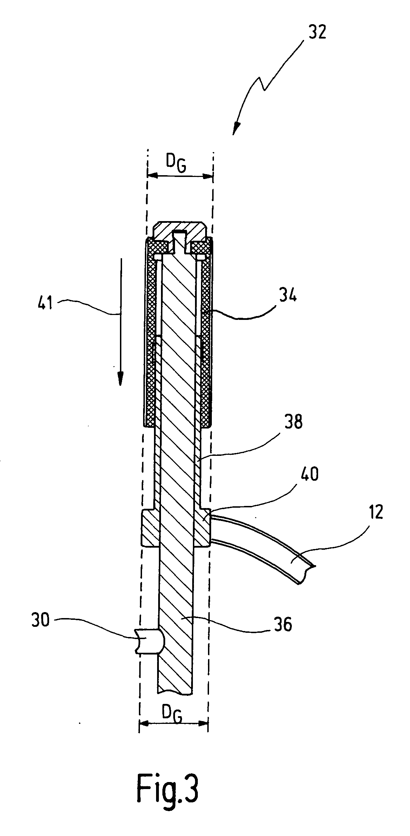

Ein detaillierter Aufbau der Verstellvorrichtung 32 ist in Fig. 3 dargestellt.

In dem Griff 34 ist ein Stab 36 aufgenommen, der mit dem Griff 34 fest verbunden ist. Der Stab 36 ist an seinem vor dem Griff 34 vorstehenden Ende mit dem Anschluss 30 fest verbunden, die in dieser Darstellung nur ansatzweise dargestellt ist.

Zwischen dem Griff 34 und dem Stab 36 ist eine Hülse 38 angeordnet.

Der Stab 36 ist über eine Längsnut 37 und einen entsprechenden Vorsprung in der Hülse 38 linear verschieblich geführt.

In der Darstellung von Fig. 3 befindet sich die Verstellvorrichtung 32 in einem teilweise vorgeschobenen Zustand, der durch den Abstand zwischen dem Griff 34 und eine Stufe 40 der Hülse 38 definiert werden kann. Wird der Griff 34 in eine Richtung gedreht, wird die Hülse 38 zwischen Griff 34 und Stab 36 geklemmt und die Verstellvorrichtung ist arretiert. Ein Drehen des Griffes 34 in die entgegengesetzte Richtung löst die Verriegelung und die Verstellvorrichtung 20 ist längs des Stabes 36 verschiebbar.

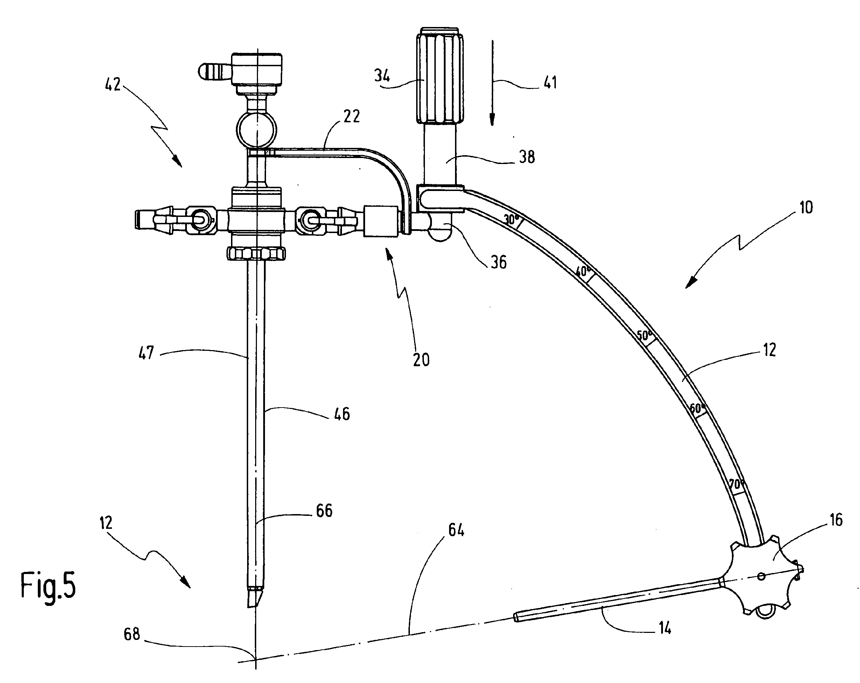

In den Figuren 4 und 5 ist dargestellt, wie das medizinische Instrument 42 an die erfindungsgemäße Teilzielvorrichtung 10 angebracht wird.

Das in Fig. 4 dargestellte medizinische Instrument 42 ist als ein Arthroskop 44 ausgebildet.

Das Arthroskop 44 weist einen Schaft 46 auf, der zur Aufnahme von einem optischen System dient. Der Schaft 46 ist mit einem Kopf 48 verbunden, der ein Gehäuse 50, und ein endseitiges Okular 52 aufweist. Zusätzlich weist es eine Spül- und Absaugvorrichtung 56 auf, an der zwei Schläuche angeschlossen werden können. Durch einen Schlauch wird Flüssigkeit in das Gelenk eingebracht, der andere dient zum Absaugen der Flüssigkeit.

Die Spül- und Absaugvorrichtung 56 weist zwei Anschlüsse 58, 60 auf, die als LUER-Anschlüsse ausgebildet sind.

Die erfindungsgemäße Teilzielvorrichtung 10 wird mit dem Arthroskop 44 an zwei Stellen verbunden. Der Anschluss 30 der Verbindungsvorrichtung 20 wird an eine bereits vorhandene Verbindungsstelle 57 des Arthroskops 44, die hier als LUER-Anschluss 58 ausgebildet ist, angebracht.

Dabei umgreift das zweite, als Gabel 28 ausgebildete Ende 26 des Stützbügels 22 die Arthroskophülse 62 und stützt sich darauf ab.

In der Darstellung von Fig. 5 ist das Arthroskop 44 mit der erfindungsgemäßen Teilzielvorrichtung 10 verbunden.

Aus dieser Darstellung ist ersichtlich, dass der Schaft 46 des Arthroskops 44 sich radial zu dem ersten Zielstab 14 erstreckt. Somit dient er als zweiter Zielstab 47.

In dem so montierten Zustand schneidet sich die Längsachse 64 des als Hülse 18 ausgebildeten ersten Zielstabs 14 und eine Längsachse 66 des Arthroskops 44 in einem Schnittpunkt 68, der der anzuzielenden Stelle entspricht.

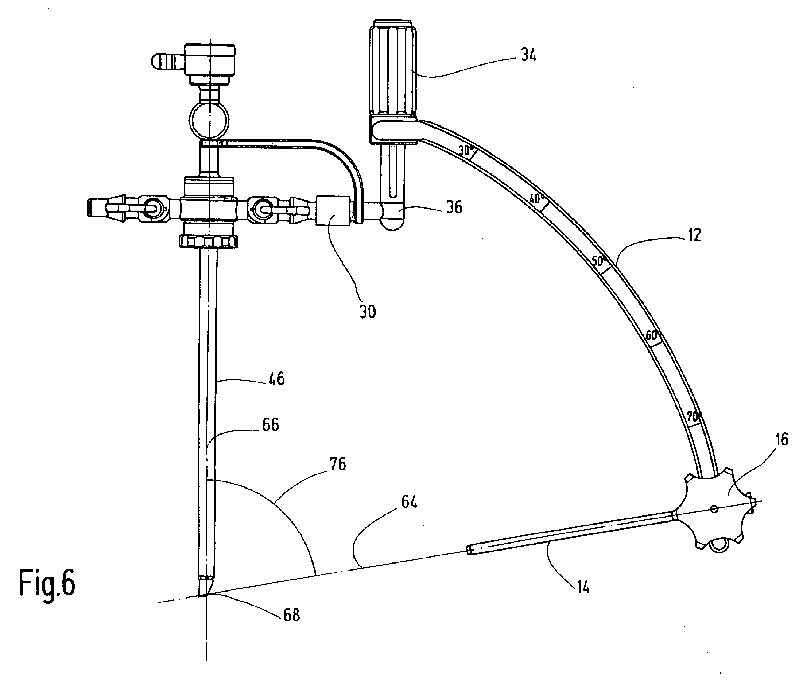

Damit ein distales Ende 72 des Schaftes 46 des Arthroskops 44 den Schnittpunkt 68 der Längsachsen 64, 68 möglichst nahe kommt, wird die Verstellvorrichtung 32 gelöst und das distale Ende des Schaftes 46 wird an das Gelenk herangelegt, und, nach Überprüfung der richtigen Position, in dieses Zielgerät eingeführt. Diese Situation ist in Fig. 6 dargestellt.

In dieser Position kann durch den als Hülse 18 ausgebildeten ersten Zielstab 14 eine Punktionskanüle eingestochen werden, die auf das distale Ende des Arthroskopschafts 46 zielt.

Nachdem die Punktionskanüle an die gewünschte Position in das Gelenk gebracht ist, wird der Schaft 46 des Arthroskops 44 ggf. unter Zuhilfenahme eines Trokardornes in das Gelenk eingestochen.

Ist das Arthroskop 44 zielgerecht an Ort und Stelle gebracht worden, kann die Teilzielvorrichtung 10 abgenommen werden. Die weiteren Manipulationen am Gelenk sind dann nicht mehr durch den Bogen 12 und den ersten Zielstab 14 gehindert.

## Patentansprüche

1. Teilzielvorrichtung (10) zum Anzielen einer arthroskopischen Operationsstelle für einen medizinischen Eingriff, mit einem kreisabschnittförmigen Bogen (12), mit einem entlang des kreisabschnittförmigen Bogens (12) bewegbaren, sich radial erstreckenden ersten Zielstab (14), wobei an dem kreisabschnittförmigen Bogen (12) eine Verbindungsvorrichtung (20) angeordnet ist, mittels der ein einen Schaft (46) aufweisendes medizinisches Instrument (42) an die Teilzielvorrichtung (10) anbringbar ist und zwar derart, dass der Schaft (46) als zweiter, radialer sich zum ersten Zielstab (14), erstreckender Zielstab (47) ausgerichtet ist, **dadurch gekennzeichnet, dass** die Verbindungsvorrichtung (20) derart ausgebildet ist, dass diese an eine bereits vorhandene, als LUER-Anschluss (58) ausgebildete Verbindungsstelle (57) des medizinischen Instruments (42) anbringbar ist.

2. Teilzielvorrichtung (10) nach Anspruch 1, **dadurch gekennzeichnet, dass** die Verbindungsvorrichtung (20) einen Stützbügel (22) zum Abstützen am medizinischen Instrument (42) aufweist.

3. Teilzielvorrichtung (10) nach Anspruch 2, **dadurch gekennzeichnet, dass** ein erstes Ende (24) des Stützbügels (22) mit der Verbindungsvorrichtung (20) fest verbunden ist.

4. Teilzielvorrichtung (10) nach Anspruch 2 oder 3, **dadurch gekennzeichnet, dass** ein zweites Ende (26) des Stützbügels (22) am medizinischen Instrument (42) anlegbar ist.

5. Teilzielvorrichtung (10) nach Anspruch 4, **dadurch gekennzeichnet, dass** das zweite Ende (26) des Stützbügels (22) am medizinischen Instrument (42) formschlüssig anlegbar ist.

6. Teilzielvorrichtung (10) nach einem der Ansprüche 1 bis 5, **dadurch gekennzeichnet, dass** die Verbindungsvorrichtung (20) zusätzlich eine Verstellvorrichtung (32) aufweist, durch die die Verbindungsvorrichtung (20) in Richtung des Schaftes (46) des medizinischen Instruments (42) axial bewegbar ist.

7. Teilzielvorrichtung (10) nach einem der Ansprüche 1 bis 6, **dadurch gekennzeichnet, dass** ein Winkel (76) zwischen einer Längsachse (64) des ersten Zielstabs (14) und einer Längsachse (66) des Schaftes (46) des medizinischen Instruments (42) etwa 30° bis 80° beträgt.

8. Teilzielvorrichtung (10) nach einem der Ansprüche 1 bis 7, **dadurch gekennzeichnet, dass** der erste Zielstab (14) als Hülse (18) ausgebildet ist.

9. Teilzielvorrichtung (10) nach einem der Ansprüche 1 bis 8, **dadurch gekennzeichnet, dass** der kreisabschnittförmige Bogen (12) eine Winkelskala (13) aufweist.

10. Teilzielvorrichtung (10) nach einem der Ansprüche 1 bis 9, **dadurch gekennzeichnet, dass** das medizinische Instrument (42) als ein Arthroskop (44) ausgebildet ist.

## Claims

1. Partial aiming device (10) for targeting an arthroscopic operation site for a medical intervention, with an arc element (12) in the shape of a section of a circle, with a first, radially extending aiming rod (14) which can be moved along the arc element (12) in the shape of a section of a circle, wherein a connecting device (20) is arranged at the arc element (12) in the shape of a section of a circle, by means of which connecting device (20), a medical instrument (42) having a shaft (46) can be connected to the partial aiming device (10) in such a way that the shaft (46) is orientated as a second, radial aiming rod (47) that extends towards the first aiming rod (14), **characterized in that** the connecting device (20) is designed **in that** it can be attached to an already existing connecting site (57) of the medical instrument (42) which is designed as a LUER-connection (58).

2. Partial aiming device (10) of claim 1, **characterized in that** the connecting device (20) has a supporting bow (22) to support the medical instrument (42).

3. Partial aiming device of claim 2, **characterized in that** a first end (24) of the supporting bow (22) is firmly connected to the connecting device (20).

4. Partial aiming device of claims 2 or 3, **characterized in that** a second end (26) of the supporting bow (22) can be brought against the medical instrument (42).

5. Partial aiming device (10) of claim 4, **characterized in that** the second end (26) of the supporting bow (22) can be brought against the medical instrument (42) in such a way as to effect a form-closure.

6. Partial aiming device (10) of any one of claims 1 to 5, **characterized in that** the connecting device (20) is in addition provided with an adjusting device (32) by means of which the connecting device (20) can be moved axially in the direction of the shaft (46) of the medical instrument (42).

7. Partial aiming device (10) of any one of claims 1 through 6, **characterized in that** an angle (76) between a longitudinal axis (64) of the first aiming rod (14) and a longitudinal axis (66) of the shaft (46) of the medical instrument (42) is approximately 30° to 80°.

8. Partial aiming device of anyone of claims 1 through 7, **characterized in that** the first aiming rod (14) is formed as a tube (18).

9. Partial aiming device of any one of claims 1 through 8, **characterized in that** the arc element (12) in the shape of a section of a circle has an angle scale (13).

10. Partial aiming device (10) of any one of claims 1 through 9, **characterized in that** the medical instrument (52) is designed as an arthroscope (44).

## Revendications

1. Dispositif partiel de visée (10) pour viser un point d'opération arthroscopique pour une intervention médicale, avec un élément (12) coudé en arc de cercle, avec une première barre de visée (14) s'étendant radialement et pouvant être déplacée le long de l'élément (12) coudé en arc de cercle, sachant qu'un dispositif de liaison (20) est disposé sur l'élément (12) coudé en arc de cercle, dispositif au moyen duquel un instrument médical (42) présentant une tige (46) peut être attaché au dispositif partiel de visée (10), et ce de telle sorte que la tige (46) est conçue comme deuxième barre de visée radiale (47) s'étendant en direction de la première barre de visée (14), **caractérisé en ce que** le dispositif de liaison (20) est conçu de telle sorte qu'il peut être mis en place en un point de liaison (57) déjà présent, conçu comme connecteur LUER (58), de l'instrument médical (42).

2. Dispositif partiel de visée (10) selon la revendication 1, **caractérisé en ce que** le dispositif de liaison (20) présente un étrier de soutien (22) pour le soutien de l'instrument médical (42).

3. Dispositif partiel de visée (10) selon la revendication 2, **caractérisé en ce qu'**une première extrémité (24) de l'étrier de soutien (22) est fixement reliée au dispositif de liaison (20).

4. Dispositif partiel de visée (10) selon la revendication 2 ou 3, **caractérisé en ce qu'**une deuxième extrémité (26) de l'étrier de soutien (22) peut être appliquée contre l'instrument médical (42).

5. Dispositif partiel de visée (10) selon la revendication 4, **caractérisé en ce que** la deuxième extrémité (26) de l'étrier de soutien (22) peut être appliquée en engagement positif contre l'instrument médical (42).

6. Dispositif partiel de visée (10) selon l'une des revendications 1 à 5, **caractérisé en ce que** le dispositif de liaison (20) présente en outre un dispositif de réglage (32), par lequel le dispositif de liaison (20) peut être déplacé axialement dans la direction de la tige (46) de l'instrument médical (42).

7. Dispositif partiel de visée (10) selon l'une des revendications 1 à 6, **caractérisé en ce qu'**un angle (76) entre un axe longitudinal (64) de la première barre de visée (14) et un axe longitudinal (66) de la tige (46) de l'instrument médical (42) est approximativement compris entre 30° et 80°.

8. Dispositif partiel de visée (10) selon l'une des revendications 1 à 7, **caractérisé en ce que** la première barre de visée (14) est réalisée sous forme de tube (18).

9. Dispositif partiel de visée (10) selon l'une des revendications 1 à 8, **caractérisé en ce que** l'élément (12) coudé en arc de cercle présente une graduation d'angle (13).

10. Dispositif partiel de visée (10) selon l'une des revendications 1 à 9, **caractérisé en ce que** l'instrument médical (42) est réalisé sous la forme d'un arthroscope (44).
